# EUROPEAN PATENT SPECIFICATION

(11) **EP 4 247 371 B1**
(45) Date of publication and mention of the grant of the patent: **01.01.2025**
(21) Application number: 21827282.1
(22) Date of filing: 18.11.2021
(51) Int. Cl.: A61K 31/4433, A61K 31/4166, A61K 45/06, A61P 35/00, A61P 35/04

(54) **EZH2 INHIBITION THERAPIES FOR THE TREATMENT OF ANDROGEN RECEPTOR MUTATED PROSTATE CANCERS**
EZH2-HEMMUNGSTHERAPIEN ZUR BEHANDLUNG VON DURCH DEN ANDROGENREZEPTOR MUTIERTEN PROSTATAKREBSEN
THÉRAPIES D'INHIBITION DE L'EZH2 POUR LE TRAITEMENT DE CANCERS DE LA PROSTATE AYANT SUBI UNE MUTATION DU RÉCEPTEUR DES ANDROGÈNES

(30) Priority: 18.11.2020 US 202063115165 P
(43) Date of publication of application: 27.09.2023
(60) Divisional application: 24206265.1
(73) Proprietor: Constellation Pharmaceuticals, Inc., Boston, MA 02210 (US)
(72) Inventor: BRADLEY, William, D., Winchester, MA 01890 (US); CUI, Jike, Belmont, MA 02478 (US); SUN, Kaiming, Belmont, MA 02478 (US); TROJER, Patrick, Reading, MA 01867 (US); WANG, Jing, Lexington, MA 20420 (US); WU, Rentian, Winchester, MA 01890 (US)
(74) Representative: Potter Clarkson
(86) International application number: PCT/US2021/059847
(87) International publication number: WO 2022/109106

(56) References cited:
- WO-A1-2019/204490
- ANANTHARAMAN ARCHANA ET AL: "Targeting the androgen receptor in metastatic castrate-resistant prostate cancer: A review", UROLOGIC ONCOLOGY: SEMINARS AND ORIGINAL INVESTIGATIONS, vol. 34, no. 8, 17 December 2015 (2015-12-17), pages 356 - 367, XP029663364, ISSN: 1078-1439, DOI: 10.1016/J.UROLONC.2015.11.003
- RICE MEGHAN A. ET AL: "Second-Generation Antiandrogens: From Discovery to Standard of Care in Castration Resistant Prostate Cancer", FRONTIERS IN ONCOLOGY, vol. 9, 28 August 2019 (2019-08-28), pages 1 - 12, XP055810336, Retrieved from the Internet <URL:https://www.ncbi.nlm.nih.gov/pmc/articles/PMC6723105/pdf/fonc-09-00801.pdf> DOI: 10.3389/fonc.2019.00801
- CLINICALTRIALS.GOV: "History of Changes for Study: NCT03480646 - ProSTAR: A Study Evaluating CPI-1205 in Patients With Metastatic Castration Resistant Prostate Cancer", CLINICALTRIALS.GOV ARCHIVE, 27 March 2018 (2018-03-27), pages 1 - 6, XP055892674, Retrieved from the Internet <URL:https://clinicaltrials.gov/ct2/history/NCT03480646?A=1&B=1&C=merged#StudyPageTop>

## Description

### RELATED APPLICATIONS

This application claims the benefit of priority to U.S. Provisional Application No. 63/115,165, filed November 18, 2020.

### BACKGROUND

According to the American Cancer Society (ACS), prostate cancer is the second most common type of cancer amongst men in the United States and is the second leading cause of cancer death in this population. The growth and survival of prostate cancer cells depend primarily on the androgen receptor signaling pathway. Cancer cells can use the binding of androgens to androgen receptors to trigger abnormal cell growth and tumor progression. The standard of care for the treatment of advanced prostate cancer is androgen deprivation therapy (ADT), which induces medical castration, or surgical castration to achieve reduced testosterone levels. Medical castration involves gonadotropin-releasing hormone antagonists, alone or in combination with first generation anti-androgen therapy. Most men with prostate cancer treated with ADT respond, as measured by tumor regression, relief of symptoms and reductions in serum prostate-specific antigen, or PSA, level and are considered to have hormone-sensitive prostate cancer.

However, almost all prostate cancer patients eventually experience a recurrence in tumor growth despite ADT. These patients are diagnosed with castration-resistant prostate cancer (CRPC), which refers to prostate cancer that progresses despite ADT and is characterized by low testosterone serum levels. The development of CRPC following ADT is due in part to tumor cells that adapt to the hormone-deprived environment of the prostate.

Castration-resistant prostate cancer that spreads, or metastasizes, to other parts of the body is diagnosed as mCRPC and may be characterized by increasing PSA levels, elevated circulating tumor cell (CTC) counts and soft tissue disease. Elevated CTC counts have been demonstrated to be a poor prognostic factor for overall survival in patients with mCRPC. See e.g., Int. J. Mol. Sci. 2016, 17(9), 1580. CTCs are cells that have shed from a primary tumor and are carried around the body in the blood and may lead to metastases. A 30% decline in CTC counts as early as four weeks after treatment initiation suggests that advanced prostate cancer patients may benefit from treatment.

Patients with mCRPC have an average survival of approximately 30 months and experience a deterioration in quality of life despite treatment with the available therapeutic options. The current standard of practice is to treat mCRPC with a second generation androgen receptor signaling (ARS) inhibitor, such as abiraterone acetate or enzalutamide. Such products are approved in the United States for first-line therapy in chemotherapy-naïve patients with mCRPC as well as for second-line treatment in patients who have received prior chemotherapy.

The problem with this treatment is that of those who have a PSA response, a large majority eventually develop resistance to ARS inhibitors. If patients with mCRPC have disease progression after treatment with a second-generation ARS inhibitor, they may be treated with either chemotherapy or a different second-generation ARS inhibitor. However, experts that treat mCRPC patients estimated that only 10-30% of patients will respond to the second-line treatment with a different ARS inhibitor and that the response achieved is typically less than half as durable, with three to six months of PSA progression-free survival, as compared to the response observed with first-line ARS inhibitor treatment. Beyond this, patients with mCRPC have very limited treatment options other than pain management and other palliative care options.

Despite these advances, the need for improved prostate cancer treatment remains.

### SUMMARY

Androgen receptor (AR) signaling is intimately involved in prostate cancer development and growth, and castration-resistant prostate cancer transformation, and is the target for hormonal therapies, such as abiraterone acetate and enzalutamide. However, AR aberrations such as AR copy number variations (CNVs), alternative splice variants, and AR point mutations are among the main causes of resistance to anti-androgen treatment. See e.g., Rice et al., Frontiers in Oncology 2019;9:1-12; Martignano et al., Transl Cancer Res 2016;5(Suppl 4):S803-S808 and Anantharaman et al., Urol Oncol 2016;34:356-67. This can lead to reduced drug efficacy and ultimately affect patient outcomes. Indeed, we have identified that subjects with prostate cancer having pathogenic androgen receptor (AR) mutations who are have been treated with enzalutamide have a lower average progression free survival while undergoing treatment with than their corresponding wild-type treated counterparts.

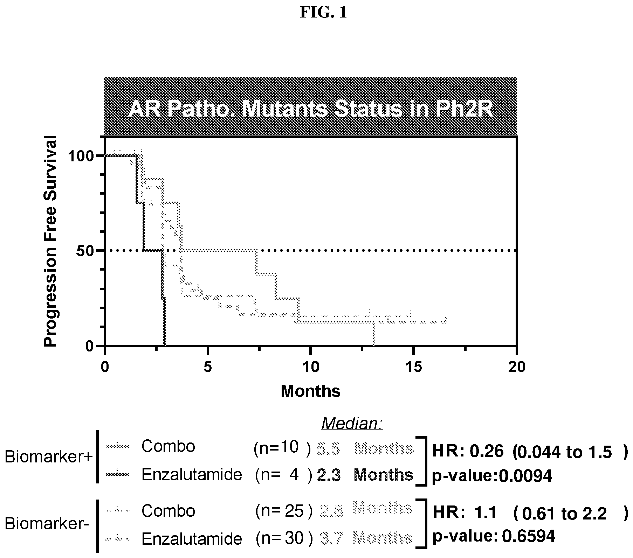

It has been found, however, that administering the EZH2 inhibitor R-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide with enzalutamide increases progression free survival in similar subjects by nearly 2.5 times. See **FIG. 1****.**

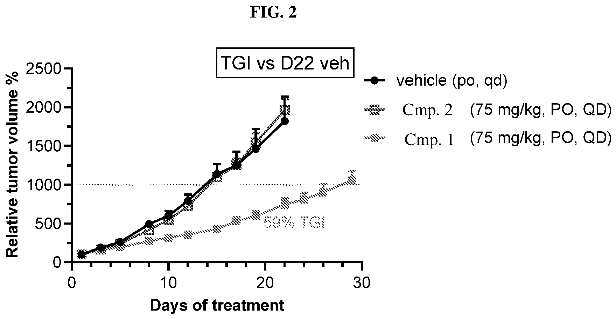

It has also been found that the EZH2 inhibitor (R)-7-chloro-2-((1r,4R)-4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is more efficacious then R-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide at reducing tumor volume in prostate cancer models with pathogenic AR mutations. See e.g., **FIG. 2****.**

Provided herein, therefore, are combinations for use in treating prostate cancer having at least one AR mutation, wherein the combination comprises a therapeutically effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof; and a therapeutically effective amount of an androgen receptor signaling inhibitor (ARSI) such as enzalutamide.

Also provided is a therapeutically effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof; and a therapeutically effective amount of an ARSI such as enzalutamide for use in treating prostate cancer characterized by having at least one AR mutation.

Also disclosed is the use of a therapeutically effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof; and a therapeutically effective amount of an ARSI such as enzalutamide in the manufacture of a medicament for treating prostate cancer characterized by having at least one AR mutation.

Also provided herein are pharmaceutical compositions comprising 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide; or a pharmaceutically acceptable salt thereof; and a second agent selected from a topoisomerase inhibitor, a DNA alkylating agent, and an ARSI; and optionally a pharmaceutically acceptable carrier, as well as said compositions for their use for treating prostate cancer characterized by having at least one AR mutation.

### BRIEF DESCRIPTION OF THE FIGURES

**FIG. 1** is a plot showing a comparison of the clinical effects from treating subjects having wild-type metastatic castration-resistant prostate cancer (mCRPC) prostate cancer and those having pathogenic AR mutated mCRPC with the combination of R-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and enzalutamide.
**FIG. 2** shows a comparison of the tumor growth inhibition (TGI), measured by relative tumor volume % reduction, between (R)-7-chloro-2-((1r,4R)-4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide and R-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide in a prostate cancer LNCaP model with AR mutations.

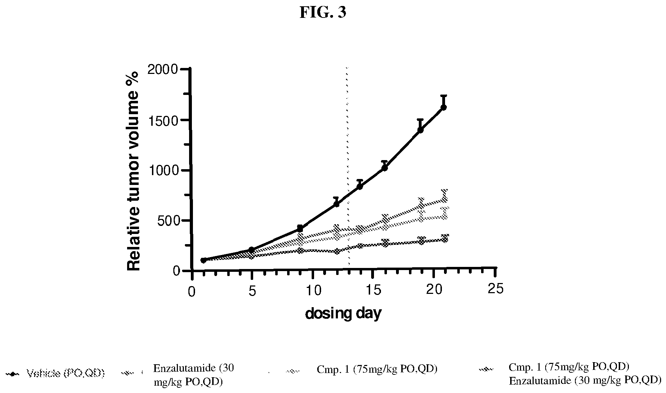

**FIG. 3** shows tumor growth inhibition (TGI), measured by relative tumor volume % reduction, in a prostate cancer LNCaP model with AR mutations using a combination of (R)-7-chloro-2-((1r,4R)-4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide and enzalutamide.

### DETAILED DESCRIPTION

The invention is as described in the appended claims. Any subject-matter falling outside the scope of the claims is provided for information only. Methods of treatment of the human body by therapy, referred to in this description, are not part of the present invention as such, but are described here in relation to compounds and pharmaceutical compositions for use in said methods of treatment of the human body by therapy, according to the present invention.

Described herein are methods of treating a prostate cancer in a subject, wherein the prostate cancer comprises at least one androgen receptor (AR) mutation, said method comprising administering to the subject an effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof. Also described herein are uses of an effective amount of 7-chloro-2-(4-(3-methoxyazetidin-1 -yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof, for the manufacture of a medicament for treating a prostate cancer comprising at least one androgen receptor (AR) mutation. According to a first embodiment provided herein is the compound 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof, for use in treating a prostate cancer comprising at least one androgen receptor (AR) mutation. Also provided as part of a first embodiment, are pharmaceutical compositions comprising an effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof, for use in treating a prostate cancer comprising at least one androgen receptor (AR) mutation.

Described herein are methods of treating a prostate cancer in a subject, wherein the prostate cancer comprises at least one androgen receptor (AR) mutation, said method comprising administering to the subject an effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof; and an effective amount of an ARSI. Also described herein are uses of an effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof; and an effective amount of an ARSI for the manufacture of a medicament for treating a prostate cancer comprising at least one androgen receptor (AR) mutation. According to a second embodiment provided herein is the compound 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof; and an effective amount of an ARSI for use in treating a prostate cancer comprising at least one androgen receptor (AR) mutation. Also provided as part of a second embodiment, are pharmaceutical compositions comprising an effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof; and an effective amount of an ARSI for use in treating a prostate cancer comprising at least one androgen receptor (AR) mutation.

In a third embodiment, the at least one AR mutation of the present invention (e.g., as in the first or second embodiment) occurs in the ligand binding domain or the transactivation domain, or both, of the AR receptor. Alternatively, as part of a third embodiment, the at least one AR mutation of the present invention (e.g., as in the first or second embodiment) occurs in the ligand binding domain. In another alternative, as part of a third embodiment, the at least one AR mutation of the present invention (e.g., as in the first or second embodiment) is selected from W742L, A491R fs, S522R, L638V, L702H, H875Y, T878A, and F877L. In another alternative, as part of a third embodiment, the at least one AR mutation of the present invention (e.g., as in the first or second embodiment) is selected from W742L, A491R fs, S522R, L638V, L702H, H875Y, and T878A. In another alternative, as part of a third embodiment, the at least one AR mutation of the present invention (e.g., as in the first or second embodiment) is a pathogenic AR mutation. Pathogenic androgen receptor (AR) mutations include those mutations that increases a subject's susceptibility or predisposition to that prostate cancer, or which decreases a subject's susceptibility, predisposition, or response to hormonal therapies such as treatment with an ARSI. Pathogenic AR mutations can occur in the ligand binding domain or the transactivation domain, or both, of the AR receptor. In another alternative, as part of a third embodiment, the at least one AR mutation of the present invention (e.g., as in the first or second embodiment) is selected from L702H, H875Y, and T878A.

In another alternative, as part of a third embodiment, the at least one AR mutation of the present invention (e.g., as in the first or second embodiment) is H875Y.

7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide and (R)-7-chloro-2-((1r,4R)-4-(3-methoxyazetidin-l-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide have the following chemical formula respectively: 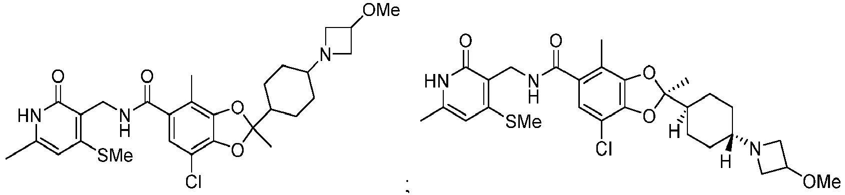 and are disclosed in international application No. PCT/US2019/027932, published as WO2019/204490. 7-chloro-2-(4-(3-methoxyazetidin-1 -yl)cyclohexyl)-2,4-dimethyl-N-((6-methyl-4-(methylthio)-2-oxo-1,2-dihydropyridin-3-yl)methyl)benzo[d][1,3]dioxole-5-carboxamide includes stereoisomeric and geometric forms. (R)-7-chloro-2-((1r,4R)-4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide as named and depicted is a single enantiomer, single geometric isomer stereochemically enriched as R about the chiral dioxolanyl carbon (e.g., by a molar excess of at least 60%, 70%, 80%, 90%, 99% or 99.9%) and geometrically enriched as a trans cyclohexyl (e.g., by a molar excess of at least 60%, 70%, 80%, 90%, 99% or 99.9%).

Androgen receptor signaling inhibitors of the present invention refer to chemical or biological agents which block the androgen receptor (AR) and inhibit or suppress androgen production. As part of a third embodiment, the androgen receptor signaling inhibitors of the present invention (e.g., as in the second embodiment) is selected from bicalutamide, enzalutamide, apalutamide, flutamide, nilutamide, darolutamide, and abiraterone acetate (wherein the abiraterone acetate may be included alone or in combination with prednisone). Alternatively, as part of a third embodiment, the androgen receptor signaling inhibitors of the present invention (e.g., as in the second embodiment) is enzalutamide. In another alternative, as part of a third embodiment, the androgen receptor signaling inhibitors of the present invention (e.g., as in the second embodiment) is abiraterone acetate (wherein the abiraterone acetate may be included alone or in combination with prednisone).

As used herein, the terms "treatment," "treat," and "treating" refer to reversing, alleviating, or inhibiting the progress of a prostate cancer, or one or more symptoms thereof, as described herein.

Prostate cancers treated by the compounds and compositions of the present invention (e.g., as in the first through third embodiments) include, but are not limited to, acinar adenocarcinoma, ductal adenocarcinoma, transitional cell carcinoma, neuroendocrine prostate cancer, basal cell prostate cancer, prostate sarcoma, and castration-resistant prostate cancer such as metastatic castration-resistant prostate cancer. Each of the disclosed prostate cancers may be "advanced", meaning that the recited cancer is unresectable, i.e., the cancer is defined as one that cannot be removed completely through surgery or that the cancer is metastatic, or both. In one aspect, "advanced cancer" means that the cancer is unresectable. In one aspect, the prostate cancer to be treated by the compounds and compositions of the present invention (e.g., as in the first through third embodiments) is metastatic castration-resistant prostate cancer

Prostate cancers described herein may also be "relapsed" cancers. The term "relapsed cancer" refers to a cancer which was previously in remission and has returned, or the signs and symptoms of the cancer have returned. Remission includes both partial remission (some or not all signs and symptoms of the cancer have disappeared) and complete remission (all signs and symptoms of the cancer have disappeared, although the cancer may still remain in the body). As such, a prostate cancer that is "advanced relapsed" means that the cancer was in remission and has returned and is unresectable.

A specific dosage and treatment regimen for any particular patient will depend upon a variety of factors, including age, body weight, general health, sex, diet, time of administration, rate of excretion, drug combination, the judgment of the treating physician, and the severity of the particular disease being treated. The amount of a provided compound in the composition will also depend upon the particular compound in the composition.

The terms "subject" and "patient" may be used interchangeably, and means a mammal in need of treatment, *e.g.,* companion animals (*e.g.,* dogs, cats, and the like), farm animals (*e.g*., cows, pigs, horses, sheep, goats and the like) and laboratory animals (*e.g*., rats, mice, guinea pigs and the like). Typically, the subject is a human in need of treatment.

The term "effective amount" or "therapeutically effective amount" refers to an amount of a compound described herein that will elicit a biological or medical response of a subject e.g., a dosage of between 0.01 - 100 mg/kg body weight/day. In one aspect, the effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof; and the effective amount of a androgen receptor signaling inhibitor described herein is such that together, they elicit a combinatorial effect to measurably treat one or more prostate cancers described herein.

Unless otherwise indicated, the administrations described herein include administering 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide prior to, concurrently with, or after administration of a disclosed androgen receptor signaling inhibitor described herein to treat a recited prostate cancer. Thus, simultaneous administration is not necessary for therapeutic purposes. In one aspect, however, 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is administered concurrently with a disclosed androgen receptor signaling inhibitor.

Further provided are pharmaceutical compositions comprising an effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof; and an effective amount of an androgen receptor signaling inhibitor; and optionally a pharmaceutically acceptable carrier.

The term "pharmaceutically acceptable carrier" refers to a non-toxic carrier, adjuvant, or vehicle that does not adversely affect the pharmacological activity of the compound with which it is formulated, and which is also safe for human use. Pharmaceutically acceptable carriers, adjuvants or vehicles that may be used in the compositions of this disclosure include, but are not limited to, ion exchangers, alumina, aluminum stearate, magnesium stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulosebased substances (e.g., microcrystalline cellulose, hydroxypropyl methylcellulose, lactose monohydrate, sodium lauryl sulfate, and crosscarmellose sodium), polyethylene glycol, sodium carboxymethylcellulose, polyacrylates, waxes, polyethylene-polyoxypropylene-block polymers, polyethylene glycol and wool fat.

The compounds described herein may be present in the form of pharmaceutically acceptable salts. For use in medicines, the salts of the compounds described herein refer to non-toxic "pharmaceutically acceptable salts." Pharmaceutically acceptable salt forms include pharmaceutically acceptable acidic/anionic or basic/cationic salts where possible.

Compositions and compounds for use herein may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques.

In one aspect, the 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide used in the disclosed uses and/or compositions is of crystalline Form **1** characterized by at least three X-ray powder diffraction peaks at 2Θ angles selected from 10.0°, 13.3°, 14.9°, 20.2°, 20.8°, 22.2°, and 22.5°. Alternatively, the 7-chloro-2-(4-(3-methoxyazetidin-1 -yl)cyclohexyl)-2,4-dimethyl-N-((6-methyl-4-(methylthio)-2-oxo-1,2-dihydropyridin-3-yl)methyl)benzo[d][1,3]dioxole-5-carboxamide used in the disclosed uses and/or compositions is of crystalline Form **1** characterized by at least four X-ray powder diffraction peaks at 2Θ angles selected from 10.0°, 13.3°, 14.9°, 20.2°, 20.8°, 22.2°, and 22.5°. In another alternative, the 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide used in the disclosed uses and/or compositions is of crystalline Form **1** characterized by at least five X-ray powder diffraction peaks at 2Θ angles selected from 10.0°, 13.3°, 14.9°, 20.2°, 20.8°, 22.2°, and 22.5°. In another alternative, the 7-chloro-2-(4-(3-methoxyazetidin-1 -yl)cyclohexyl)-2,4-dimethyl-N-((6-methyl-4-(methylthio)-2-oxo-1,2-dihydropyridin-3-yl)methyl)benzo[d][1,3]dioxole-5-carboxamide used in the disclosed uses and/or compositions is of crystalline Form **1** characterized by at least six X-ray powder diffraction peaks at 2Θ angles selected from 10.0°, 13.3°, 14.9°, 20.2°, 20.8°, 22.2°, and 22.5°. In another alternative, the 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide used in the disclosed uses and/or compositions is of crystalline Form **1** characterized by X-ray powder diffraction peaks at 2Θ angles selected from 10.0°, 13.3°, 14.9°, 20.2°, 20.8°, 22.2°, and 22.5°. In another alternative, the 7-chloro-2-(4-(3-methoxyazetidin-1 -yl)cyclohexyl)-2,4-dimethyl-N-((6-methyl-4-(methylthio)-2-oxo-1,2-dihydropyridin-3-yl)methyl)benzo[d][1,3]dioxole-5-carboxamide used in the disclosed uses and/or compositions is of crystalline Form **1** characterized by X-ray powder diffraction peaks at 2Θ angles selected from 10.0°, 10.2°, 12.3°, 12.7°, 13.3°, 14.9°, 15.3°, 20.2°, 20.8°, 21.3°, 22.2°, 22.5°, and 23.8°. In another alternative, the 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide used in the disclosed uses and/or compositions is of crystalline Form **1** characterized by X-ray powder diffraction peaks at 2Θ angles selected from 10.0°, 10.2°, 11.0°, 11.4°, 11.8°, 12.3°, 12.7°, 13.3°, 14.9°, 15.3°, 16.1°, 17.4°, 20.2°, 20.8°, 21.3°, 22.2°, 22.5°, and 23.8°. In another alternative, the 7-chloro-2-(4-(3-methoxyazetidin-1 -yl)cyclohexyl)-2,4-dimethyl-N-((6-methyl-4-(methylthio)-2-oxo-1,2-dihydropyridin-3-yl)methyl)benzo[d][1,3]dioxole-5-carboxamide used in the disclosed uses and/or compositions is of crystalline Form **1** characterized by x-ray powder diffraction peaks at 2Θ angles selected from 14.9°, 20.2°, and 20.8°. In another alternative, the 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide used in the disclosed uses and/or compositions is of crystalline Form **1** characterized by x-ray powder diffraction peaks at 2Θ angles selected from 10.0°, 14.9°, 20.2°, and 20.8°. In another alternative, the 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide used in the disclosed uses and/or compositions is of crystalline Form **1** characterized by x-ray powder diffraction peaks at 2Θ angles selected from 10.0°, 14.9°, 20.2°, 20.8°, and 22.2°. In another alternative, 7-chloro-2-(4-(3-methoxyazetidin-l-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is of crystalline Form **1** characterized by x-ray powder diffraction peaks at 2Θ angles selected from 10.0°, 13.3°, 14.9°, 20.2°, 20.8°, and 22.2°. Polymorphic forms of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide and salts thereof are described in PCT/US2020/043178.

In one aspect, (R)-7-chloro-2-((1r,4R)-4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is used in the present uses and compositions.

### EXEMPLIFICATION

(R)-7-chloro-2-((1r,4R)-4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide can be prepared following the procedures described in PCT/US2019/027932 (WO2019/204490) and PCT/US2020/043163.

### Progression Free Survival Effects from Co-Administration of R-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and Enzalutamide in Prostate Cancer Subjects

Phase 2 clinical studies were conducted using R-N-((4-methoxy-6-methyl-2-oxo-1,2-dihydropyridin-3-yl)methyl)-2-methyl-1 -(1 -(1 -(2,2,2-trifluoroethyl)piperidin-4-yl)ethyl)-1H-indole-3-carboxamide / enzalutamide combination versus enzalutamide alone in second-line mCRPC subjects. See e.g., ClinicalTrials.gov Identifier: NCT03480646. Tumors samples from the individuals in that trial were studied post-treatment. Of the subjects treated, 9.4% had pathogenic mutations L702H, 12.5% had pathogenic mutations T878A, and 9.4% had pathogenic mutations H875Y.

It was found that subjects with who were treated with R-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide / enzalutamide combination and had tumors characterized by the pathogenic mutations above, had a higher average progression free survival than subjects who had wild-type AR tumors, i.e., no pathogenic mutations in the AR domain. These results are shown in **FIG. 1**, where Biomarker + patients are those who have at least one pathogenic mutation selected from L702H, T878A, and H875Y. Combo refers to use of R-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide with enzalutamide.

### (R)-7-chloro-2-((1r,4R)-4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide Shows Greater Efficacy in Prostate Cancer LNCaP Model with AR Mutation H875Y

(R)-7-chloro-2-((1r,4R)-4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide and R-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide were examined in a prostate cancer LNCaP Model with AR mutation H875Y.

A fixed dose of 75 mg/kg was used for both compounds. Each mouse was inoculated subcutaneously at the right flank with LNCaP-FGC tumor cells (10 × 10⁶) in 0.1 ml of PBS mixed with RPMI-1640:Matrigel (50:50) for tumor development. The treatments started when the average tumor volume reached approximately 158 mm³. The test articles were administered orally once daily. The tumor size was used for the calculation of tumor growth inhibition (TGI) = [1 - (T1-T0)/(C1-C0)] x 100, where C1= mean tumor volume of control mice at time t, T1 = mean tumor volume of treated mice at time t, C0 = mean tumor volume of control mice at time 0, and T0 = mean tumor volume of treated mice at time 0].

As shown by the data in **FIG. 2**, (R)-7-chloro-2-((1r,4R)-4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide (labeled as Cmp. 1), was more efficacious at tumor growth inhibition then R-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide (labeled as Cmp. 2). Thus, its use in treating prostate cancers with AR mutations in combination with ARSIs like enzalutamide is conceivable.

### Synergistic effects from Co-Administration of (R)-7-chloro-2-((1r,4R)-4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide and Enzalutamide in Prostate Cancer LNCaP Model with AR Mutation T878A

A fixed dose of 75 mg/kg was used for (R)-7-chloro-2-((1r,4R)-4-(3-methoxyazetidin-1 -yl)cyclohexyl)-2,4-dimethyl-N-((6-methyl-4-(methylthio)-2-oxo-1,2-dihydropyridin-3-yl)methyl)benzo[d][1,3]dioxole-5-carboxamide (labeled as Cmp. 1) and 30 mg/kg was used for enzalutamide. Each mouse was inoculated subcutaneously at the right flank with LNCaP-FGC tumor cells (10 × 10⁶) in 0.1 ml of PBS mixed with RPMI-1640:Matrigel (50:50) for tumor development. The treatments started when the average tumor volume reached approximately 158 mm³. The test articles were administered orally once daily. The tumor size was used for the calculation of tumor growth inhibition (TGI) = [1 - (T1-T0)/(C1-C0)] x 100, where C1= mean tumor volume of control mice at time t, T1 = mean tumor volume of treated mice at time t, C0 = mean tumor volume of control mice at time 0, and T0 = mean tumor volume of treated mice at time 0]. As shown by the data in **FIG. 3**, combination treatment with Cmp. 1 and enzalutamide was more effective at inhibiting tumor growth than the use of Cmp. 1 or enzalutamide alone.

Unless otherwise defined, all technical and scientific terms used herein are accorded the meaning commonly known to one with ordinary skill in the art.

## Claims

1. A compound having the formula: 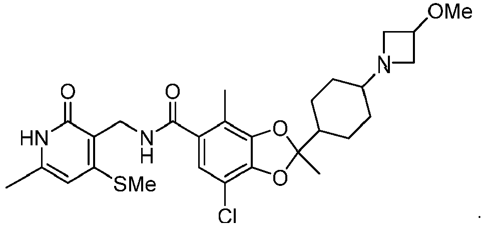
or a pharmaceutically acceptable salt thereof, for use in the treatment of a prostate cancer,
wherein the prostate cancer comprises at least one pathogenic androgen receptor mutation.

2. The compound for use of Claim 1, wherein the treatment further comprises administering an effective amount of an androgen receptor signaling inhibitor.

3. The compound for use of Claim 2, wherein the androgen receptor signaling inhibitor is selected from bicalutamide, enzalutamide, apalutamide, flutamide, nilutamide, darolutamide, and abiraterone acetate.

4. The compound for use of Claim 2 or 3, wherein the androgen receptor signaling inhibitor is enzalutamide.

5. The compound for use of any one of Claims 1 to 4, wherein the prostate cancer is metastatic castration-resistant prostate cancer (mCRPC).

6. The compound for use of any one of Claims 1 to 5, wherein the at least one pathogenic androgen receptor mutation occurs in the ligand binding domain or transactivation domain of the androgen receptor.

7. The compound for use of any one of Claims 1 to 6, wherein the at least one pathogenic androgen receptor mutation occurs in the ligand binding domain.

8. The compound for use of any one of Claims 1 to 6, wherein the at least one androgen receptor mutation is selected from L702H, H875Y, and T878A.

9. The compound for use of any one of Claims 1 to 7, wherein the at least one pathogenic androgen receptor mutation is H875Y.

10. The compound for use of any one of Claims 1 to 9, wherein the compound is of the formula 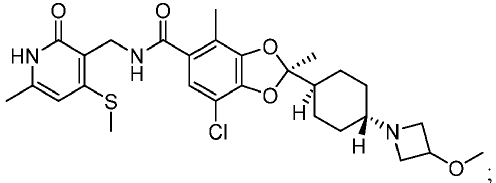 or a pharmaceutically acceptable salt thereof.

11. A pharmaceutical composition comprising a compound having the formula: 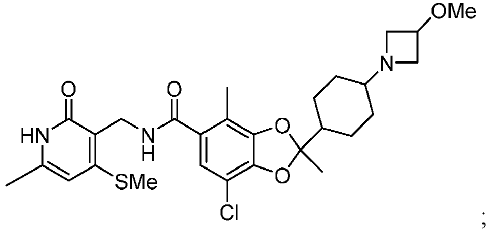 or a pharmaceutically acceptable salt thereof; and an effective amount of an androgen receptor signaling inhibitor.

12. The pharmaceutical composition of Claim 11, wherein the androgen receptor signaling inhibitor is selected from bicalutamide, enzalutamide, apalutamide, flutamide, nilutamide, darolutamide, and abiraterone acetate.

13. The pharmaceutical composition of Claim 11 or 12, wherein the androgen receptor signaling inhibitor is enzalutamide.

14. The pharmaceutical composition of any one of Claims 11 to 13, wherein the compound is of the formula 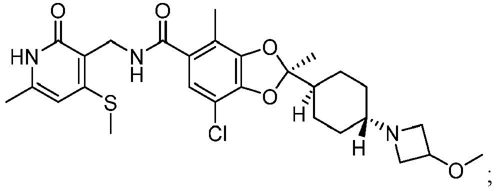 or a pharmaceutically acceptable salt thereof.

## Patentansprüche

1. Verbindung, die die folgende Formel aufweist: 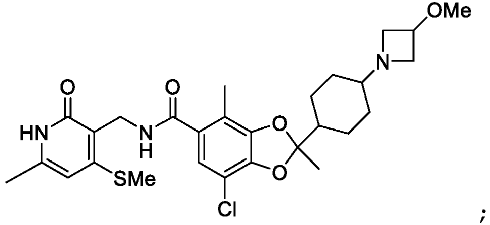 oder ein pharmazeutisch verträgliches Salz davon, zur Verwendung bei der Behandlung von Prostatakrebs, wobei der Prostatakrebs mindestens eine pathogene Androgenrezeptormutation umfasst.

2. Verbindung zur Verwendung nach Anspruch 1, wobei die Behandlung ferner die Verabreichung einer wirksamen Menge eines Androgenrezeptor-Signalisierungsinhibitors umfasst.

3. Verbindung zur Verwendung nach Anspruch 2, wobei der Androgenrezeptor-Signalisierungsinhibitor ausgewählt ist aus Bicalutamid, Enzalutamid, Apalutamid, Flutamid, Nilutamid, Darolutamid und Abirateronacetat.

4. Verbindung zur Verwendung nach Anspruch 2 oder 3, wobei der Androgenrezeptor-Signalisierungsinhibitor Enzalutamid ist.

5. Verbindung zur Verwendung nach einem der Ansprüche 1 bis 4, wobei der Prostatakrebs ein metastasierter kastrationsresistenter Prostatakrebs (mCRPC) ist.

6. Verbindung zur Verwendung nach einem der Ansprüche 1 bis 5, wobei die mindestens eine pathogene Androgenrezeptormutation in der Ligandenbindungsdomäne oder Transaktivierungsdomäne des Androgenrezeptors auftritt.

7. Verbindung zur Verwendung nach einem der Ansprüche 1 bis 6, wobei die mindestens eine pathogene Androgenrezeptormutation in der Ligandenbindungsdomäne auftritt.

8. Verbindung zur Verwendung nach einem der Ansprüche 1 bis 6, wobei die mindestens eine Androgenrezeptormutation ausgewählt ist aus L702H, H875Y und T878A.

9. Verbindung zur Verwendung nach einem der Ansprüche 1 bis 7, wobei die mindestens eine pathogene Androgenrezeptormutation H875Y ist.

10. Verbindung zur Verwendung nach einem der Ansprüche 1 bis 9, wobei die Verbindung die folgende Formel aufweist 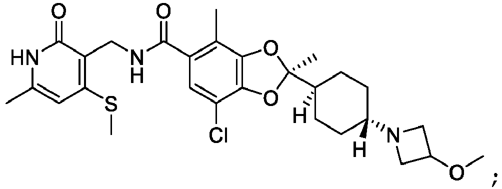 oder ein pharmazeutisch verträgliches Salz davon.

11. Pharmazeutische Zusammensetzung, umfassend eine Verbindung mit der Formel: 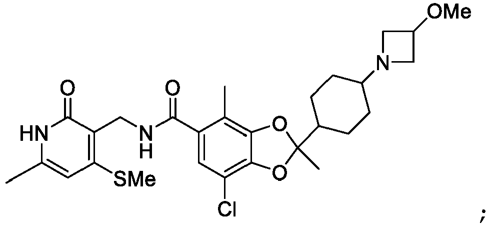 oder ein pharmazeutisch verträgliches Salz davon; und eine wirksame Menge eines Androgenrezeptor-Signalisierungsinhibitors.

12. Pharmazeutische Zusammensetzung nach Anspruch 11, wobei der Androgenrezeptor-Signalisierungsinhibitor ausgewählt ist aus Bicalutamid, Enzalutamid, Apalutamid, Flutamid, Nilutamid, Darolutamid und Abirateronacetat.

13. Pharmazeutische Zusammensetzung nach Anspruch 11 oder 12, wobei der Androgenrezeptor-Signalisierungsinhibitor Enzalutamid ist.

14. Pharmazeutische Zusammensetzung nach einem der Ansprüche 11 bis 13, wobei die Verbindung die folgende Formel aufweist 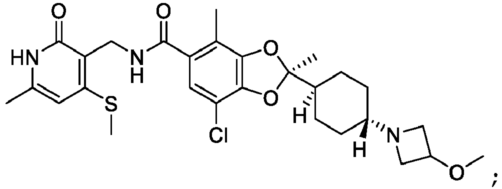 oder ein pharmazeutisch verträgliches Salz davon.

## Revendications

1. Composé présentant la formule : 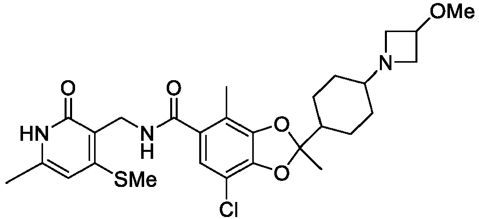 ou sel pharmaceutiquement acceptable de celui-ci, pour utilisation dans le traitement d'un cancer de la prostate, dans lequel le cancer de la prostate comprend au moins une mutation pathogène du récepteur d'androgènes.

2. Composé pour utilisation selon la revendication 1, dans lequel le traitement comprend en outre l'administration d'une quantité efficace d'un inhibiteur de signalisation du récepteur d'androgènes.

3. Composé pour utilisation selon la revendication 2, dans lequel l'inhibiteur de signalisation du récepteur d'androgènes est sélectionné parmi le bicalutamide, l'enzalutamide, l'apalutamide, le flutamide, le nilutamide, le darolutamide, et l'acétate d'abiratérone.

4. Composé pour utilisation selon la revendication 2 ou 3, dans lequel l'inhibiteur de signalisation du récepteur d'androgènes est l'enzalutamide.

5. Composé pour utilisation selon l'une quelconque des revendications 1 à 4, dans lequel le cancer de la prostate est un cancer de la prostate résistant à la castration métastatique (mCRPC).

6. Composé pour utilisation selon l'une quelconque des revendications 1 à 5, dans lequel l'au moins une mutation pathogène du récepteur d'androgènes apparaît dans le domaine de liaison au ligand ou dans le domaine de transactivation du récepteur d'androgènes.

7. Composé pour utilisation selon l'une quelconque des revendications 1 à 6, dans lequel l'au moins une mutation pathogène du récepteur d'androgènes apparaît dans le domaine de liaison au ligand.

8. Composé pour utilisation selon l'une quelconque des revendications 1 à 6, dans lequel l'au moins une mutation du récepteur d'androgènes est sélectionnée parmi L702H, H875Y et T878A.

9. Composé pour utilisation selon l'une quelconque des revendications 1 à 7, dans lequel l'au moins une mutation pathogène du récepteur d'androgènes est H875Y.

10. Composé pour utilisation selon l'une quelconque des revendications 1 à 9, dans lequel le composé est de la formule 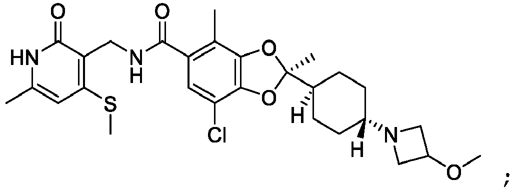 ou sel pharmaceutiquement acceptable de celui-ci.

11. Composition pharmaceutique comprenant un composé présentant la formule : 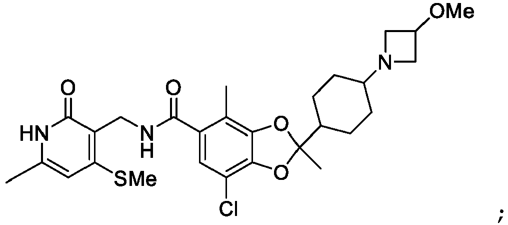 ou un sel pharmaceutiquement acceptable de celui-ci ; et une quantité efficace d'un inhibiteur de signalisation du récepteur d'androgènes.

12. Composition pharmaceutique selon la revendication 11, dans laquelle l'inhibiteur de signalisation du récepteur d'androgènes est sélectionné parmi le bicalutamide, l'enzalutamide, l'apalutamide, le flutamide, le nilutamide, le darolutamide, et l'acétate d'abiratérone.

13. Composition pharmaceutique selon la revendication 11 ou 12, dans laquelle l'inhibiteur de signalisation du récepteur d'androgènes est l'enzalutamide.

14. Composition pharmaceutique selon l'une quelconque des revendications 11 à 13, dans laquelle le composé est de la formule 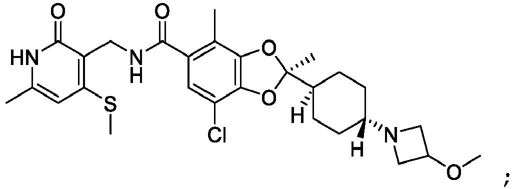 ou un sel pharmaceutiquement acceptable de celui-ci.
